# EUROPÄISCHE PATENTSCHRIFT

(11) **EP 1 567 075 B1**
(45) Veröffentlichungstag und Bekanntmachung des Hinweises auf die Patenterteilung: **27.11.2013**
(21) Anmeldenummer: 02782597.5
(22) Anmeldetag: 06.12.2002
(51) Int. Cl.: A61B 17/80

(54) **VORRICHTUNG FÜR DIE OSTEOSYNTHESE**
DEVICE FOR OSTEOSYNTHESIS
DISPOSITIF POUR L'OSTEOSYNTHÈSE

(43) Veröffentlichungstag der Anmeldung: 31.08.2005
(73) Patentinhaber: Synthes GmbH, 4436 Oberdorf (CH)
(72) Erfinder: MATHIEU, Claude, CH-8002 Zurich (CH); FRIGG, Robert, CH-2544 Bettlach (CH); SCHNEIDER, Rolf, CH-2562 Port (CH); APPENZELLER, Andreas, CH-2504 Biel (CH)
(74) Vertreter: Lusuardi, Werther
(86) Internationale Anmeldenummer: PCT/CH2002/000673
(87) Internationale Veröffentlichungsnummer: WO 2004/052219

(56) Entgegenhaltungen:
- EP-A- 0 337 288
- WO-A-98/25534
- WO-A-03/055401
- DE-A- 19 720 782

## Beschreibung

Die Erfindung betrifft eine Vorrichtung für die Osteosynthese gemäss dem Oberbegriff des Patentanspruchs 1.

Solche Vorrichtungen werden zur polyaxialen, rigiden Verschraubung verwendet, insbesondere im Bereich der Wirbelsäule, beispielsweise für Pedikelschrauben oder Pedikelhaken. Sie können aber auch generell zur Plattenosteosynthese verwendet werden. Zudem sind auch Anwendungen für Fixateur externes sowie für Zwischenwirbelimplantate möglich.

Aus der US-A 6,235,033 ist bereits eine derartige Vorrichtung bekannt, bei der zwischen dem Schraubenkopf und der Bohrung der Knochenplatte ein schwenkbarer, ringförmiger Einsatz vorhanden ist, welcher mittels eines Schlitzes komprimierbar und expandierbar ist, um damit eine verbesserte Fixation zwischen Schraube und Platte zu erreichen. Die Nachteile dieser bekannten Vorrichtung sind vielfältig:
- der kreisrund ausgebildete, axial starre Einsatz kann sich beim Schraubeneindrehen mitdrehen und verhindert dadurch das Verriegeln;
- der Einsatz kann sich aber auch ganz innerhalb der Plattenbohrung verdrehen, so dass er dann mit der falschen Seite (Innenkonus verjüngt sich in die falsche Richtung) nach oben zu liegen kommt.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Hier will die Erfindung Abhilfe schaffen. Der Erfindung liegt das Problem zugrunde, eine Vorrichtung für die Osteosynthese zu schaffen, bei welcher die longitudinalen Knochenfixationselemente (im folgenden am Beispiel von Knochenschrauben näher ausgeführt) relativ zur Knochenplatte polyaxial beweglich und winkelstabil verriegelbar sind und welche insgesamt nur 4 Konstruktionselemente erfordert, nämlich ein Knochenplatte, einen Einsatz für das Plattenloch, ein longitudinales Knochenfixationselement (z.B. Knochenschraube) und ein Eindrehinstrument (z.B. einen Schraubendreher.

Die Erfindung löst die gestellte Aufgabe mit einer Vorrichtung, welche die Merkmale des Anspruchs 1 aufweist. Dank seiner Ausbildung als Spiralfeder bleibt der im Durchgang des Osteosynthese-Elementes einsetzbare Einsatz relativ zu Letzterem in mehreren Richtungen d.h. multiaxial beweglich, bzw. schwenkbar. Diese Schwenkbarkeit ist im Vergleich zum sphärischen Einsatz der US 6, 235, 033 nur eine teilweise. Gegenüber der US 6,235,033 besitzt die Erfindung den Vorteil, dass keine zusätzliche Spreizschraube notwendig ist. Vorteilhaft ist auch, dass die naturgemäss axial komprimierbare und expandierbare Spiralfeder gleichzeitig auch radial komprimierbar und expandierbar ist. Zudem ist die Herstellung vereinfacht und billiger. Die Spiralfeder hat weiter den Vorteil, dass sich beim Eindrehen einer Knochenschraube, der - vorzugsweise konisch ausgebildete - Kopf der Knochenschraube im Inneren der Spiralfeder verkeilt.

Bei einer bevorzugten Ausführungsform sind die Windungen (vorzugsweise 2 oder mehr Windungen) der Spiralfeder unrund ausgebildet. Als "unrund" wird im folgenden jeder von der exakt kreisförmigen Fläche abweichende Querschnitt bezeichnet, insbesondere prismatische und elliptische Querschnitte. Auch der orthogonal zur Zentralachse stehende Querschnitt des Durchgangs ist vorzugsweise unrund ausgebildet. Der orthogonal zur Längsachse stehende Querschnitt der äusseren Hüllfläche der Spiralfeder weist dabei vorteilhafterweise eine zum Querschnitt des Durchgangs im wesentlichen korrespondierende Form auf. Damit ist der zusätzliche Vorteil erzielbar, dass sich der Einsatz beim Einschrauben der Knochenschraube nicht mehr um seine eigene Achse drehen kann. Letzteres würde nämlich dazu führen, dass keine Relativbewegung mehr zwischen Einsatz und Schraube stattfände, so dass auch kein Aufspreizen des Einsatzes mehr möglich wäre und damit auch keine Verriegelung der Schraube.

Die Rotationsblockierung des Einsatzes im Durchgang kann nicht nur durch eine entsprechende Geometrie des Durchgangs und des Einsatzes erreicht werden, sondern auch mit anderen Mitteln, welche dazu führen, dass der Einsatz relativ zu seiner Längsachse rotationsfest im Durchgang gelagert ist, solange nur der Einsatz innerhalb des Durchgangs relativ zum Osteosynthese-Element mindestens teilweise axial verschiebbar und vorzugsweise schwenkbar bleibt. Beispielsweise genügt es schon wenn die Spiralfeder an mindestens einem Punkt ihrer Federwendel (z.B. an einem ihrer freien Enden) im Durchgang befestigt ist.

Bei einer besonderen Ausführungsform ist der Querschnitt des Durchgangs in der vorzugsweise als Knochenplatte ausgebildeten osteosynthetischen Vorrichtung polygonal, vorzugsweise hexagonal, ausgebildet, so dass der Durchgang ein Prisma, vorzugsweise ein sechsseitiges Prisma darstellt. Die Spiralfeder weist dann vorteilhafterweise ebenfalls einen polygonalen (z.B. hexagonalen) Querschnitt auf. Bei der hexagonalen Ausführung lässt sich die Knochenschraube im sechskantigen Durchgang gleichzeitig in drei Ebenen schwenken, so dass jeder beliebige Schwenkwinkel einstellbar ist. Dieser ist nur durch die Plattendicke und durch das Anstossen des Einsatzes an der vorteilhafterweise angebrachten Querschnittsverengung begrenzt. Bei den meisten Knochenplatten sind natürlich mehrere Durchgänge vorhanden.

Bei einer weiteren Ausführungsform erweitert sich der Durchgang des Osteosynthese-Elementes mindestens in Richtung einer seiner beiden Austrittsöffnungen, vorzugsweise konisch. Der Durchgang des Einsatzes ist typischerweise prismatisch, vorzugsweise als sechsseitiges Prisma ausgebildet. Er kann aber auch hohlkreiszylinderförmig ausgebildet sein.

Bei einer weiteren Ausführungsform ist der Durchmesser der zentralen Bohrung des Einsatzes in einer Richtung verjüngt und die Bohrung vorzugsweise als Konus augebildet. Diese Ausgestaltung erlaubt eine Aufspreizung des Einsatzes mittels eines Gegenkonus, d.h. zum Beispiel mit einer Knochenschraube, welche einen konischen Schraubenkopf aufweist. Die Bohrung des Einsatzes kann aber auch kreiszylindrisch ausgebildet sein.

Der orthogonal zur Zentralachse stehende Querschnitt des Durchgangs in der vorzugsweise als Knochenplatte ausgebildeten osteosynthetischen Vorrichtung kann auch ellipsenförmig ausgebildet sein.

Die Oberfläche des Einsatzes, vorzugsweise im Bereich seiner äusseren Hüllkurve, ist zweckmässigerweise aufgerauht, z.B. rauh gestrahlt. Entsprechend kann der Durchgang in der Knochenplatte aufgerauht, z.B. rauh gestrahlt sein.
Der Durchgang kann dann in entsprechender Weise mit einer Makrostrukturierung versehen sein, z.B. in Form peripher umlaufender Rillen. Der Vorteil dieser Ausgestaltung liegt in der damit erzielten formschlüssigen Verbindung zwischen Einsatz und Knochenplatte.
Die Oberfläche des Einsatzes kann, vorzugsweise im Bereich seiner äusseren Hüllkurve, mit einer Beschichtung aus einem härteren Material als der Einsatz versehen sein und ev. zusätzlich mit einer Makrostrukturierung versehen sein. Der Durchgang des Osteosynthese-Elementes kann ebenfalls aufgerauht sein, vorzugsweise rauh gestrahlt sein. Der Durchgang des Osteosynthese-Elementes kann mit einer Beschichtung aus einem härteren Material als dasjenige des Osteosynthese-Elementes versehen sein.

Bei einer weiteren speziellen Ausführungsform verjüngt sich der Durchgang in der vorzugsweise als Knochenplatte ausgebildeten osteosynthetischen Vorrichtung gegen die Unterseite hin und vorzugsweise auch gegen die Oberseite hin, so dass eine Querschnittsverengung resultiert, welche das Herausfallen oder Herausdrücken des Einsatzes verhindert. Zweckmässigerweise werden dabei die Querschnittsverengung des Durchgangs und die Komprimierbarkeit des Einsatzes derart aufeinander abgestimmt, dass der als Spiralfeder ausgebildete Einsatz durch temporäre Verringerung seines grössten Durchmessers in den Durchgang einbringbar ist.

Bei einer besonderen Ausführungsform ist die äussere Hüllfläche des Einsatzes konvex und der Durchgang des Osteosynthese-Elementes konkav ausgebildet.

Vorteilhafterweise besteht die osteosynthetische Vorrichtung, mindestens im Bereich ihres Durchganges und der Einsatz, ebenfalls mindestens im Bereich seiner äusseren Hüllkurve, aus verschiedenen Materialien, vorzugsweise solchen mit unterschiedlicher Härte. Der Einsatz kann beispielsweise aus einem biokompatiblen Kunststoff bestehen und die osteosynthetische Vorrichtung (z.B. eine Knochenplatte) aus einem körperverträglichen Metall. Der Einsatz kann aber auch metallisch sein und die Vorrichtung aus einem Kunststoff, vorzugsweise einem verstärkten Kunststoff. Die unterschiedlichen Materialien bewirken eine plastische Verformung der Oberflächen und dadurch einen Formschluss.

Die Höhe des Einsatzes sollte in Richtung seiner Längsachse gemessen, kleiner sein als die Höhe des Durchgangs in der Knochenplatte in Richtung seiner Zentralachse gemessen. Die Höhe des Einsatzes liegt zweckmässigerweise im Bereich von 40 % - 85 %, vorzugsweise von 45 % bis 65 % der Höhe des Durchgangs.

Die zur Einführung in den Einsatz dienenden Knochenschrauben weisen vorzugsweise einen konischen Schraubenkopf auf, der zudem mit einem Aussengewinde versehen sein kann. Der Vorteil dieser Ausgestaltung liegt darin, dass das Spreizen und Verriegeln des Einsatzes in einem einzigen Schritt ermöglicht wird.

Bei einer besonderen Ansführungsform ist der Durchmesser des Federdrahtes der Spiralfeder kleiner oder gleich gross ist als der Abstand zwischen zwei Gewindespitzen des Aussengewindes des Schraubenkopfes. Der Querschnitt des Federdrahtes der Spiralfeder kann auch unrund, vorzugsweise quadratisch oder rautenförmig sein.

Die Steigung der Windungen der Spiralfeder sollte vorteilhafterweise im wesentlichen der Steigung des Aussengewindes des Schraubenkopfes entsprechen. Der Durchmesser des Federdrahtes der Spiralfeder sollte zudem vorteilhafterweise grösser sein als die radiale Querschnittsverengung des Durchgangs.

Die Erfindung und Weiterbildungen der Erfindung werden im folgenden anhand der teilweise schematischen Darstellungen mehrerer Ausführungsbeispiele noch näher erläutert. Alle Ausführungsbeispiele beziehen sich auf eine Knochenplatte als osteosynthetische Vorrichtung. Analoge Anwendungen für Pedikelschrauben, Pedikelhaken, Fixateur externes oder Zwischenwirbelimplantate sind möglich.

Es zeigen:
Fig. 1 eine perspektivische Darstellung einer in Form einer Knochenplatte realisierten Vorrichtung für die Osteosynthese;
Fig. 2 einen Querschnitt durch die Knochenplatte nach Fig. 1 mit einem eingesetzten Einsatz in Form einer Spiralfeder;
Fig. 3 einen Querschnitt längs der Linie III-III in Fig. 2. 1;
Fig. 4 ein schematische, perspektivische Ansicht eines Einsatzes in Form einer hexagonalen Spiralfeder;
Fig. 5 eine perspektivische Darstellung der Knochenplatte nach Fig. 1 mit dem eingesetzten Einsatz nach Fig. 4; und
Fig. 6 einen Längsschnitt durch eine Knochenschraube für die Vorrichtung für die Osteosynthese.

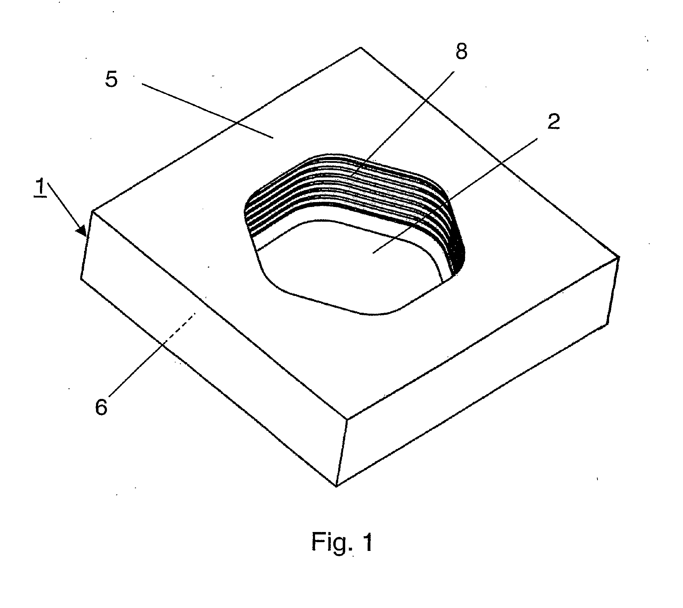

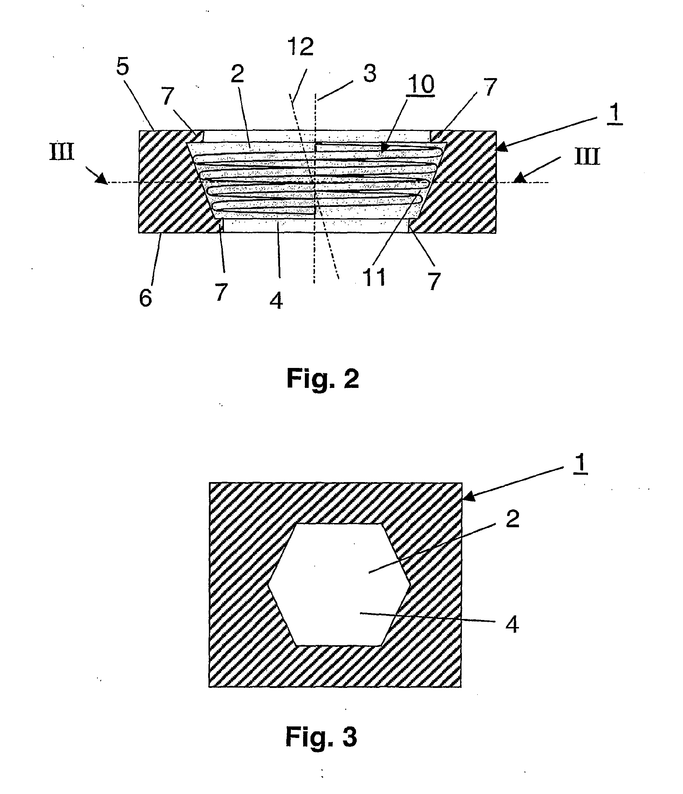

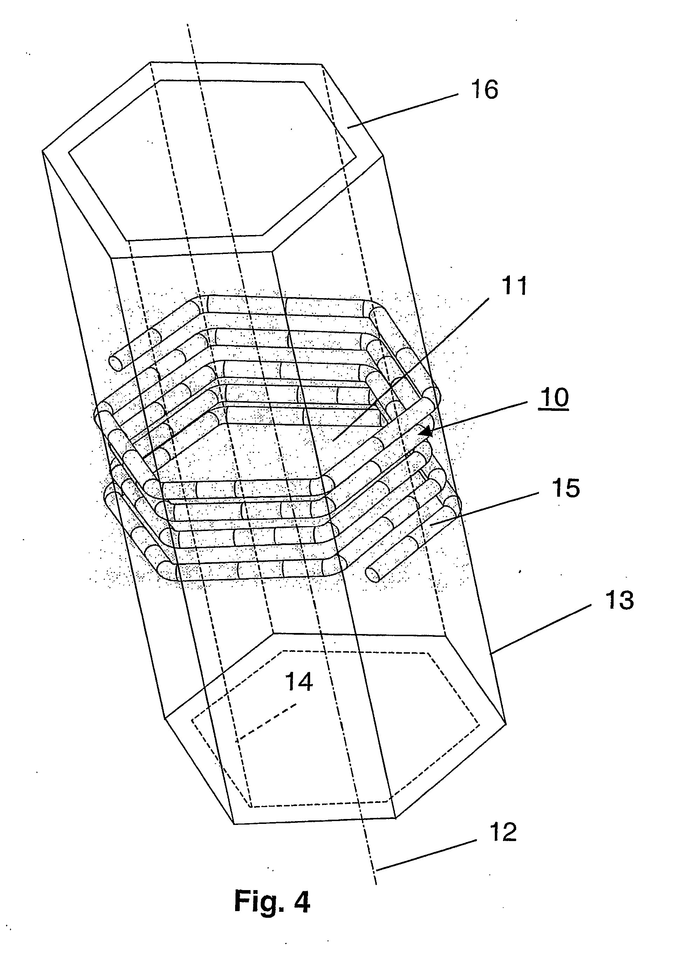

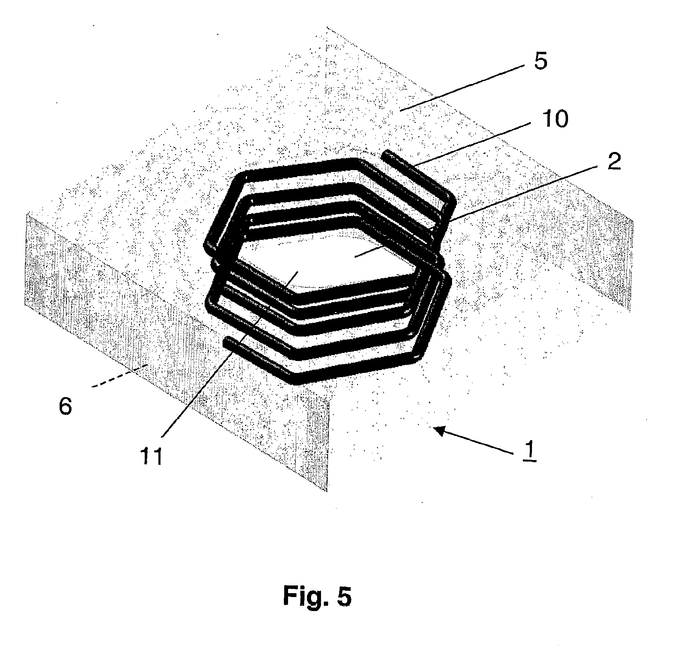

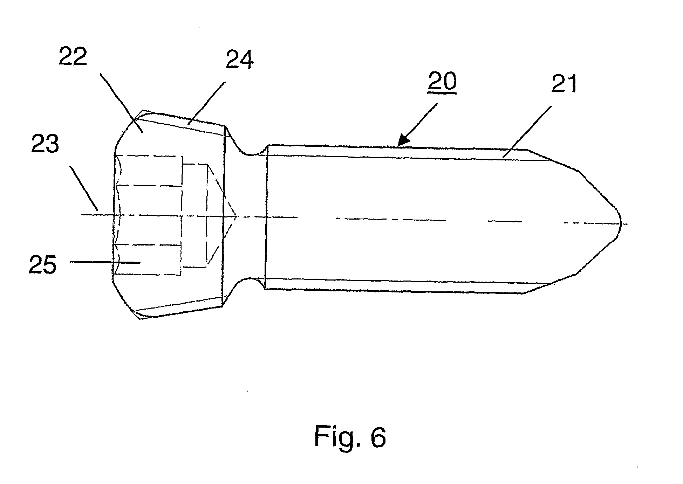

Die in den Fig. 1 bis 5 dargestellte Vorrichtung für die Osteosynthese besteht aus einer Knochenplatte 1 mit einer für die Anlage zum Knochen bestimmten Unterseite 6, einer Oberseite 5, sowie einem die Unterseite 6 mit der Oberseite 5 verbindenden Durchgang 2 zur Aufnahme eines multiaxial schwenkbaren Einsatzes 10 (Fig. 2 und 4) für eine Knochenschraube 20 (Fig. 6), wobei der Durchgang 2 eine Zentralachse 3 aufweist. Der in den Durchgang 2 einsetzbare Einsatz 10 (Fig. 2 und 4) besitzt einen zentralen Durchgang 11 zur Aufnahme der Knochenschraube 20 (Fig. 6), wobei der zentrale Durchgang 11 eine Längsachse 12 aufweist, eine äussere Hüllfläche 13, welche für den Kontakt mit dem Durchgang 2 bestimmt ist und eine, den zentralen Durchgang 11 definierende, innere Hüllfläche 14.

Der Einsatz 10 besteht aus einer hexagonalen Spiralfeder, so dass er radial komprimierbar und radial expandierbar ist. Der Durchgang 2 der Knochenplatte 1 weist gegen die Unterseite 6 und auch gegen die Oberseite 5 hin, eine radiale Querschnittsverengung 7 auf, um das Herausfallen oder Herausdrücken des Einsatzes 10 zu verhindern. Die Querschnittsverengung 7 des Durchgangs 2 und die Komprimierbarkeit des Einsatzes 10 sind derart aufeinander abgestimmt, dass der Einsatz 10 im komprimierten Zustand in den Durchgang 2 einbringbar ist.

Wie in Fig. 1 gezeigt ist der Durchgang 2 der Knochenplatte 1 mit einer Makrostrukturierung in Form peripher umlaufender Rillen 8 versehen.

Wie in Fig. 3 gezeigt, ist der orthogonal zur Zentralachse 3 stehende Querschnitt 4 des Durchgangs 2 annähernd hexagonal, d.h. unrund ausgebildet ist. Der orthogonal zur Längsachse 12 stehende Querschnitt des Einsatzes 10 weist eine zum Querschnitt 4 des Durchganges 2 der Knochenplatte 1 im wesentlichen korrespondierende Form auf, so dass der im Durchgang 2 eingesetzte Einsatz 10 relativ zu seiner Längsachse 12 rotationsfest ist, aber innerhalb des Durchgangs 2 relativ zur Knochenplatte 1 teilweise axial beweglich, bzw. schwenkbar bleibt.
Wie in Fig. 2 dargestellt, verjüngt sich der Durchmesser des Durchgangs 4 in Richtung der Unterseite 6 der Knochenplatte 1 hin, so dass der Durchgang 4 konisch augebildet ist. Entsprechend ist die den Einsatz 10 bildende Spiralfeder ebenfalls konisch ausgebildet.

In den Einsatz 10 kann die in Fig. 6 dargestellte Knochenschraube 20 eingesetzt werden. Die Knochenschraube 20 besitzt einen zur Verankerung im Knochen bestimmten Gewindeschaft 21, eine Schraubenachse 23, sowie einen zur Einführung in den zentralen Durchgang 11 des Einsatzes 10 bestimmten Schraubenkopf 22, welcher im wesentlichen der Form des zentralen Durchgangs 11 entspricht. Der orthogonal zur Schraubenachse 23 stehende Querschnitt durch den Schraubenkopf 22 ist gegen den Gewindeschaft 21 hin verjüngt, so dass ein Konus resultiert. Der Schraubenkopf 22 ist mit einem Aussengewinde 24 versehen, dessen Steigung mit der Steigung der Windungen der als Einsatz 10 realisierten Spiralfeder übereinstimmt. Im weiteren besitzt der Schraubenkopf 22 einen Innensechskant 25 zur Aufnahme eines (zeichnerisch nicht dargestellten) Sechskantschraubendrehers.

Im folgenden wird die klinische Verwendung der Vorrichtung für die Osteosynthese kurz beschrieben.
Der Einsatz 10 der Vorrichtung ist bereits im Durchgang 2 der Knochenplatte 1 oder einer anderen osteosynthetischen Vorrichtung vormontiert. Ein Einsetzen durch den Chirurgen entfällt somit. Die Knochenplatte 1 wird mit den in den Durchgängen 2 vormontierten Einsätzen 10 auf den Knochen gelegt. Dies kann entweder vor oder auch nach dem Reponieren der verschiedenen Bruchstücke, bzw. Wirbelkörper geschehen. Zum Setzen der Knochenschrauben 20 gibt es drei Standard-Szenarien:
a) Bohren, Gewindeschneiden, Schrauben;
b) Bohren, Schrauben (mit selbstschneidenden Schrauben); oder
c) Schrauben (mit selbstbohrenden und selbstschneidenden Schrauben).
Auch die Verwendung von Ziel-, bzw. Bohrbuchsen ist möglich. Natürlich ist die Benützung von festen Zielbuchsen nicht sinnvoll, weil dadurch der Vorteil einer winkelverstellbaren Schraube verloren ginge, hingegen kann eine solche Zielbuchse sinnvoll sein, um den Verstellbereich einzuschränken. Bohrbuchsen kommen dann zum Einsatz wenn keine selbstbohrenden Schrauben verwendet werden und zuerst ein Loch gebohrt werden muss. In einem solchen Fall dient die Bohrbuchse dem Schutz der Weichteile.

Beim Setzen mehrerer Knochenschrauben 20 gibt es grundsätzlich zwei Möglichkeiten:
A) Im Fall, dass die Reposition vor dem Anlegen der Knochenplatte gemacht wird, können die Knochenschrauben 20 sofort fixiert werden; und
B) für den Fall, dass die Reposition nach dem Anlegen der Knochenplatte gemacht wird, werden die Knochenschrauben 20 nur soweit eingedreht, damit die Knochenplatte am Knochen fixiert ist; erst dann findet die endgültige Reposition oder Korrektur statt und die Knochenschrauben 20 können durch Weiterdrehen um wenige Winkelgrade blockiert werden.

## Patentansprüche

1. Vorrichtung für die Osteosynthese mit
A) einem Osteosynthese-Element (1) mit einer oberen Seite (5) und einer unteren Seite (6) und einem, die obere und untere Seite (5,6) verbindenden Durchgang (2) zur Aufnahme eines relativ zum Osteosynthese-Element (1) multiaxial schwenkbaren Einsatzes (10) für ein longitudinales Knochenfixationselement (20), wobei der Durchgang (2) eine Zentralachse (3) aufweist;
B) einem in den Durchgang (2) einsetzbaren Einsatz (10) mit einem zentralen Durchgang (11) zur Aufnahme des Knochenfixations-Elementes (20), wobei der Durchgang (11) eine Längsachse (12) aufweist,
**dadurch gekennzeichnet, dass**
C) der Einsatz (10) als Spiralfeder mit einer äusseren Hüllfläche (13) und einer, den zentralen Durchgang (11) definierenden, inneren Hüllfläche (14) ausgebildet ist.

2. Vorrichtung nach Anspruch 1, **dadurch gekennzeichnet, dass** die Windungen (15) der Spiralfeder unrund, vorzugsweise prismatisch ausgebildet sind.

3. Vorrichtung nach Anspruch 1 oder 2, **dadurch gekennzeichnet, dass** der orthogonal zur Zentralachse (3) stehende Querschnitt (4) des Durchgangs (2) unrund, vorzugsweise prismatisch ausgebildet ist.

4. Vorrichtung nach einem der Ansprüche 1 bis 3, **dadurch gekennzeichnet, dass** der orthogonal zur Längsachse (12) stehende Querschnitt (16) der äusseren Hüllfläche (13) der Spiralfeder (10) eine zum Querschnitt (4) des Durchgangs (2) im wesentlichen korrespondierende Form aufweist.

5. Vorrichtung nach einem der Ansprüche 1 bis 4, **dadurch gekennzeichnet, dass** die Spiralfeder radial komprimierbar beziehungsweise expandierbar ist.

6. Vorrichtung nach einem der Ansprüche 1 bis 5, **dadurch gekennzeichnet, dass** der im Durchgang (2) eingesetzte Einsatz (10) relativ zu seiner Längsachse (12) rotationsfest gelagert ist, aber innerhalb des Durchgangs (2) relativ zum Osteosynthese-Element (1) mindestens teilweise axial verschiebbar und/oder schwenkbar bleibt.

7. Vorrichtung nach einem der Ansprüche 1 bis 6, **dadurch gekennzeichnet, dass** der Querschnitt (4) des Durchgangs (2) polygonal, vorzugsweise hexagonal, ausgebildet ist.

8. Vorrichtung nach einem der Ansprüche 1 bis 7, **dadurch gekennzeichnet, dass** der Durchgang (2) im wesentlichen prismatisch, vorzugsweise als sechsseitiges Prisma ausgebildet ist.

9. Vorrichtung nach einem der Ansprüche 1 bis 8, **dadurch gekennzeichnet, dass** sich der Durchgang (2) des Osteosynthese-Elementes (1) mindestens in Richtung einer seiner beiden Austrittsöffnungen, vorzugsweise konisch erweitert.

10. Vorrichtung nach einem der Ansprüche 1 bis 9, **dadurch gekennzeichnet, dass** sich der zentrale Durchgang (11) des Einsatzes (10) mindestens in Richtung einer seiner beiden Austrittsöffnungen, vorzugsweise konisch erweitert.

11. Vorrichtung nach einem der Ansprüche 1 bis 10, **dadurch gekennzeichnet, dass** der zentrale Durchgang (11) des Einsatzes (10) prismatisch, vorzugsweise als sechsseitiges Prisma ausgebildet ist.

12. Vorrichtung nach einem der Ansprüche 1 bis 10, **dadurch gekennzeichnet, dass** der zentrale Durchgang (11) des Einsatzes (10) hohlkreiszylinderförmig ist.

13. Vorrichtung nach einem der Ansprüche 1 bis 12, **dadurch gekennzeichnet, dass** die Oberfläche des Einsatzes (10), vorzugsweise im Bereich seiner äusseren Hüllkurve (13), aufgerauht ist, vorzugsweise rauh gestrahlt ist.

14. Vorrichtung nach einem der Ansprüche 1 bis 13, **dadurch gekennzeichnet, dass** die Oberfläche des Einsatzes (10), vorzugsweise im Bereich seiner äusseren Hüllkurve (13), mit einer Beschichtung aus einem härteren Material als der Einsatz (10) versehen ist.

15. Vorrichtung nach einem der Ansprüche 1 bis 14, **dadurch kennzeichnet, dass** die Oberfläche des Einsatzes (10), vorzugsweise im Bereich seiner äusseren Hüllkurve (13), mit einer Makrostrukturierung versehen ist.

16. Vorrichtung nach einem der Ansprüche 1 bis 15, **dadurch gekennzeichnet, dass** der Durchgang (2) des Osteosynthese-Elementes (1) aufgerauht ist, vorzugsweise rauh gestrahlt ist.

17. Vorrichtung nach einem der Ansprüche 1 bis 16, **dadurch gekennzeichnet, dass** der Durchgang (2) des Osteosynthese-Elementes (1) mit einer Beschichtung aus einem härteren Material als dasjenige des Osteosynthese-Elementes (1) versehen ist.

18. Vorrichtung nach einem der Ansprüche 1 bis 17, **dadurch gekennzeichnet, dass** der Durchgang (2) des Osteosynthese-Elementes (1) mit einer Makrostrukturierung versehen ist, vorzugsweise in Form von peripher umlaufenden Rillen (8).

19. Vorrichtung nach einem der Ansprüche 1 bis 18, **dadurch gekennzeichnet, dass** der Durchgang (2) des Osteosynthese-Elementes (1) gegen seine untere Seite (6) hin und vorzugsweise auch gegen seine obere Seite (5) hin, eine Querschnittsverengung (7) aufweist, um das Herausfallen oder Herausdrücken des Einsatzes (10) zu verhindern.

20. Vorrichtung nach Anspruch 19, **dadurch gekennzeichnet, dass** die Querschnittsverengung (7) des Durchgangs (2) und der als Spiralfeder ausgebildete Einsatz (10) derart aufeinander abgestimmt sind dass der Einsatz (10) durch temporäre Verringerung seines grössten Durchmessers in den Durchgang (4) einbringbar ist.

21. Vorrichtung nach einem der Ansprüche 1 bis 20, **dadurch gekennzeichnet, dass** die äussere Hüllfläche (13) des Einsatzes (10) konvex ausgebildet ist.

22. Vorrichtung nach einem der Ansprüche 1 bis 21, **dadurch gekennzeichnet, dass** der Durchgang (2) des Osteosynthese-Elementes (1) konkav ausgebildet ist.

23. Vorrichtung nach einem der Ansprüche 1 bis 22, **dadurch gekennzeichnet, dass**
A) das Osteosynthese-Element (1), mindesten im Bereich seines Durchgangs (2) und
B) der Einsatz (10), mindestens im Bereich seiner äusseren Hüllfläche (13); aus
C) verschiedenen Materialien, vorzugsweise solchen mit unterschiedlicher Härte, bestehen.

24. Vorrichtung nach einem der Ansprüche 1 bis 23, **dadurch gekennzeichnet, dass** das Osteosynthese-Element (1) aus einem Kunststoff, vorzugsweise einem verstärkten Kunststoff besteht und der Einsatz (10) metallisch ist.

25. Vorrichtung nach einem der Ansprüche 1 bis 24, **dadurch gekennzeichnet, dass** die Höhe des Einsatzes (10) in Richtung seiner Längsachse (12) gemessen, kleiner ist als die Höhe des Durchgangs (2) in Richtung seiner Zentralachse (3) gemessen.

26. Vorrichtung nach Anspruch 25, **dadurch gekennzeichnet, dass** die Höhe des Einsatzes (10) im Bereich von 40 % - 85 %, vorzugsweise von 45 % bis 65 % der Höhe des Durchgangs (2) liegt.

27. Vorrichtung nach einem der Ansprüche 1 bis 26, **dadurch gekennzeichnet, dass** sie ein longitudinales Knochenfixationselement (20) umfasst, vorzugsweise in Form einer Knochenschraube mit einem zur Verankerung im Knochen bestimmten Gewindeschaft (21), einer Schraubenachse (23), und einem zur Einführung in den zentralen Durchgang (11) des Einsatzes (10) bestimmten Schraubenkopf (22), welcher im wesentlichen der Form des Durchgangs (11) des Einsatzes (10) entspricht.

28. Vorrichtung nach Anspruch 27, **dadurch gekennzeichnet, dass** sich der orthogonal zur Schraubenachse (23) stehende Querschnitt durch den Schraubenkopf (22) gegen den Gewindeschaft (21) hin verjüngt, vorzugsweise in Form eines Konus.

29. Vorrichtung nach Anspruch 27 oder 28, **dadurch gekennzeichnet, dass** der Schraubenkopf (22) mit einem Aussengewinde (24) versehen ist.

30. Vorrichtung nach einem der Ansprüche 1 bis 29, **dadurch gekennzeichnet, dass**
A) das Osteosynthese-Element (1) eine Knochenplatte ist; und
B) die untere Seite (6) für die Anlage zum Knochen geeignet ist.

31. Vorrichtung nach einem der Ansprüche 1 bis 29, **dadurch gekennzeichnet, dass** das Osteosynthese-Element (1) eine Pedikelschraube oder ein Pedikelhaken ist.

32. Vorrichtung nach einem der Ansprüche 1 bis 29, **dadurch gekennzeichnet, dass** das Osteosynthese-Element (1) ein Fixateur externe ist.

33. Vorrichtung nach einem der Ansprüche 1 bis 29, **dadurch gekennzeichnet, dass** das Osteosynthese-Element (1) ein Zwischenwirbelimplantat ist.

34. Vorrichtung nach einem der Ansprüche 29 bis 33, **dadurch gekennzeichnet, dass** der Durchmesser des Federdrahtes der als Einsatz (10) realisierten Spiralfeder kleiner oder gleich gross ist als der Abstand zwischen zwei Gewindespitzen des Aussengewindes (24) des Schraubenkopfes (22).

35. Vorrichtung nach einem der Ansprüche 1 bis 34, **dadurch gekennzeichnet, dass** der Querschnitt des Federdrahtes der als Einsatz (10) realisierten Spiralfeder unrund, vorzugsweise quadratisch oder rautenförmig ist.

36. Vorrichtung nach einem der Ansprüche 29 bis 35, **dadurch gekennzeichnet, dass** die Steigung der Windungen des als Einsatz (10) realisierten Spiralfeder im wesentlichen der Steigung des Aussengewindes (24) des Schraubenkopfes (22) entspricht.

37. Vorrichtung nach einem der Ansprüche 19 bis 36, **dadurch gekennzeichnet, dass** der Durchmesser des Federdrahtes der Spiralfeder grösser ist als die radiale Querschnittsverengung (7) des Durchgangs (2).

## Claims

1. Device for osteosynthesis with
A) an osteosynthesis element (1) with a top side (5) and an underside (6) and a passage (2) connecting the top side (5) with the underside (6) for receiving multi-axially tiltable relative to the osteosynthesis element (1) an insert (10) for a longitudinal bone fixation element (20), wherein the passage (2) has a central axis (3);
B) an insert (10) insertable in the passage (2) with a central passage (11) for receiving the bone fixation element (20), wherein the passage (11) has a longitudinal axis (12),
**characterized in that**
C) the insert (10) is configured as a coil spring with an external enveloping surface (13) and an internal enveloping surface (14) defining the central passage (11).

2. Device according to claim 1, **characterized in that** the windings (15) of the coil spring are configured noncircularly, preferably prismatically.

3. Device according to claim 1, **characterized in that** the cross-section (4) of the passage (2) standing orthogonally to the central axis (3) is configured noncircularly, preferably prismatically.

4. Device according to one of claims 1 to 3, **characterized in that** the cross-section (16) of the external enveloping surface (13) of the coil spring (10) standing orthogonally to the longitudinal axis (12) is of a form generally corresponding to the cross-section (4) of the passage (2).

5. Device according to one of claims 1 to 4, **characterized in that** the coil spring is radially compressable, respectively expandable.

6. Device according to one of claims 1 to 5, **characterized in that** the insert (10) inserted in the passage (2) is carried rotatively fixed relative to its longitudinal axis (12) but remains within the passage (2) at least partially axially moveable or tiltable relative to the osteosynthesis element (1).

7. Device according to one of claims 1 to 6, **characterized in that** the cross-section (4) of the passage (2) is designed in polygonal and preferably in hexagon form.

8. Device according to one of claims 1 to 7, **characterized in that** the passage (2) is designed generally prismatic, and preferably as a hexagonal prism.

9. Device according to one of claims 1 to 8, **characterized in that** the passage (2) of the osteosynthesis element (1) extends preferably conically at least in the direction of one of its two outlet openings.

10. Device according to one of claims 1 to 9, **characterized in that** the central passage (11) of the insert (10) extends preferably conically at least in the direction of one of its two outlet openings.

11. Device according to one of claims 1 to 10, **characterized in that** the central passage (11) of the insert (10) is designed prismatically, and preferably as a hexagonal prism.

12. Device according to one of claims 1 to 10, **characterized in that** the central passage (11) of the insert (10) has the form of a circular hollow cylinder.

13. Device according to one of claims 1 to 12, **characterized in that** the surface of the insert (10) is roughened preferably in the area of its external enveloping surface (13), and preferably blasted rough.

14. Device according to one of claims 1 to 13, **characterized in that** the surface of the insert (10) is provided with a coating made of a material harder than that used for the insert (10) preferably in the area of its external enveloping surface (13).

15. Device according to one of claims 1 to 14, **characterized in that** the surface of the insert (10) is provided with macro-structuring preferably in the area of its external enveloping surface (13).

16. Device according to one of claims 1 to 15, **characterized in that** the passage (2) of the osteosynthesis element (1) is roughened, and preferably blasted rough.

17. Device according to one of claims 1 to 16, **characterized in that** the passage (2) of the osteosynthesis element (1) is provided with a coating made of a material harder than that used for the osteosynthesis element (1) itself.

18. Device according to one of claims 1 to 17, **characterized in that** the passage (2) of the osteosynthesis element (1) is provided with macro-structuring, preferably in the form of grooves (8) running around the periphery.

19. Device according to one of claims 1 to 18, **characterized in that** the passage (2) of the osteosynthesis element (1) is provided with a narrowing (7) of the cross-section towards its underside (6) and preferably also towards its top side (5) in order to prevent the insert (19) from falling for from being pushed out.

20. Device according to claim 19, **characterized in that** the cross-section narrowing (7) of the passage (2) and the insert (10) formed as a coil spring are co-ordinated so that the insert (10) can be inserted into the passage (4) by a temporary reduction of its largest diameter.

21. Device according to one of claims 1 to 20, **characterized in that** the external enveloping surface (13) of the insert (10) is convexly designed.

22. Device according to one of claims 1 to 21, **characterized in that** the passage (2) of the osteosynthesis element (1) is concavely designed.

23. Device according to one of claims 1 to 22, **characterized in that**
A) the osteosynthesis element (1), at least in the area of its passage (2), and
B) the insert (10), at least in the area of its external enveloping surface (13),
C) are made of different materials, preferably materials with different degrees of hardness.

24. Device according to one of claims 1 to 23, **characterized in that** the osteosynthesis element (1) is made of a synthetic material, preferably of a reinforced plastic, and the insert (10) is metallic.

25. Device according to one of claims 1 to 24, **characterized in that** the height of the insert (10) is, when measured in the direction of its longitudinal axis (12), smaller than the height of the passage (2) measured in the direction of its central axis (3).

26. Device according to one of claim 25, **characterized in that** the height of the insert (10) is within the range of 40% to 85%, preferably of 45% to 65%, of the height of the passage (2).

27. Device according to one of claims 1 to 26, **characterized in that** it comprises a longitudinal bone fixation element (20), preferably in the form of a bone screw, with a threaded shaft (21) intended for anchoring in the bone, a screw axis (23) and a screw head 22 intended for insertion in the central passage (11) of the insert (10), which corresponds generally to the form of the passage (11) of the insert (10).

28. Device according to one of claim 27, **characterized in that** the cross-section of the screw head (22) orthogonal to the screw axis (23) tapers towards the threaded shaft (21), preferably in the form of a cone.

29. Device according to claim 27 or 28, **characterized in that** the screw head (22) is provided with an external thread (24).

30. Device according to one of claims 1 to 29, **characterized in that**
A) the osteosynthesis element (1) is a bone plate, and
B) the underside (6) is suitable for contact to the bone.

31. Device according to one of claims 1 to 29, **characterized in that** the osteosynthesis element (1) is a pedicle screw or a pedicle hook.

32. Device according to one of claims 1 to 29, **characterized in that** the osteosynthesis element (1) is an external fixator.

33. Device according to one of claims 1 to 29, **characterized in that** the osteosynthesis element (1) is an intervertebral implant.

34. Device according to one of claims 29 to 33, **characterized in that** the diameter of the spring wire of the coil spring realised as an insert (10) is smaller than or of the same size as the distance between two threading tips of the external thread (24) of the screw head (22).

35. Device according to one of claims 1 to 34, **characterized in that** the cross-section of the spring wire of the coil spring realised as an insert (10) is noncircular, preferably square or rhombic.

36. Device according to one of claims 29 to 35, **characterized in that** the pitch of the windings of the coil spring realised as an insert (10) generally corresponds to the pitch of the external thread (24) of the screw head (22).

37. Device according to one of claims 19 to 36, **characterized in that** the diameter of the spring wire of the coil spring is larger than the radial cross-section narrowing (7) of the passage (2).

## Revendications

1. Dispositif d'ostéosynthèse, avec
A) un élément d'ostéosynthèse (1) avec un côté supérieur (5) et un côté inférieur (6) et un passage (2) raccordant le côté supérieur et le côté inférieur (5, 6) pour la réception d'un insert (10), pivotant de façon multiaxiale par rapport à l'élément d'ostéosynthèse (1), pour un élément de fixation osseuse (20) longitudinal, le passage (2) présentant un axe central (3) ;
B) un insert (10) pouvant être inséré dans le passage (2), avec un passage (11) central pour la réception de l'élément de fixation osseuse (20), le passage (11) présentant un axe longitudinal (12),
**caractérisé en ce que**
C) l'insert (10) est réalisé en tant que ressort en spirale avec une surface enveloppante extérieure (13) et une surface enveloppante intérieure (14) définissant le passage (11) central.

2. Dispositif selon la revendication 1, **caractérisé en ce que** les spires (15) du ressort en spirale sont réalisées de façon non ronde, de préférence de façon prismatique.

3. Dispositif selon la revendication 1 ou 2, **caractérisé en ce que** la section transversale (4) du passage (2) qui est orthogonale à l'axe central (3) est réalisée de façon non ronde, de préférence de façon prismatique.

4. Dispositif selon une des revendications 1 à 3, **caractérisé en ce que** la section transversale (16) de la surface enveloppante extérieure (13) du ressort à spirale (10) qui est orthogonale à l'axe longitudinal (12) présente une forme qui correspond essentiellement à la section transversale (4) du passage (2).

5. Dispositif selon une des revendications 1 à 4, **caractérisé en ce que** le ressort à spirale peut être comprimé ou respectivement dilaté dans le sens radial.

6. Dispositif selon une des revendications 1 à 5, **caractérisé en ce que** l'insert (10) inséré dans le passage (2) est supporté de façon fixe en rotation par rapport à son axe longitudinal (12), mais continue à pouvoir, au moins partiellement, se déplacer et/ou pivoter dans le sens axial par rapport à l'élément d'ostéosynthèse (1).

7. Dispositif selon une des revendications 1 à 6, **caractérisé en ce que** la section transversale (4) du passage (2) est réalisée de façon polygonale, de préférence hexagonale.

8. Dispositif selon une des revendications 1 à 7, **caractérisé en ce que** le passage (2) est réalisé de façon essentiellement prismatique, de préférence en tant que prisme à six côtés.

9. Dispositif selon une des revendications 1 à 8, **caractérisé en ce que** le passage (2) de l'élément d'ostéosynthèse (1) est, au moins en direction d'une de ses deux ouvertures de sortie, élargi de préférence de façon conique.

10. Dispositif selon une des revendications 1 à 9, **caractérisé en ce que** le passage (11) central de l'insert (10) est, au moins en direction d'une de ses deux ouvertures de sortie, élargi de préférence de façon conique.

11. Dispositif selon une des revendications 1 à 10, **caractérisé en ce que** le passage (11) central de l'insert (10) est réalisé de façon prismatique, de préférence en tant que prisme à six côtés.

12. Dispositif selon une des revendications 1 à 10, **caractérisé en ce que** le passage (11) central de l'insert (10) est en forme de cylindre circulaire creux.

13. Dispositif selon une des revendications 1 à 12, **caractérisé en ce que** la surface de l'insert (10), de préférence dans la zone de son enveloppante extérieure (13), est rendue rugueuse, de préférence par grenaillage.

14. Dispositif selon une des revendications 1 à 13, **caractérisé en ce que** la surface de l'insert (10), de préférence dans la zone de son enveloppante extérieure (13), est munie d'un revêtement en matériau plus dur que l'insert (10).

15. Dispositif selon une des revendications 1 à 14, **caractérisé en ce que** la surface de l'insert (10), de préférence dans la zone de son enveloppante extérieure (13), est munie d'une macrostructuration.

16. Dispositif selon une des revendications 1 à 15, **caractérisé en ce que** le passage (2) de l'élément d'ostéosynthèse (1) est rendu rugueux, de préférence par grenaillage.

17. Dispositif selon une des revendications 1 à 16, **caractérisé en ce que** le passage (2) de l'élément d'ostéosynthèse (1) est muni d'un revêtement en matériau plus dur que celui de l'élément d'ostéosynthèse (1).

18. Dispositif selon une des revendications 1 à 17, **caractérisé en ce que** le passage (2) de l'élément d'ostéosynthèse (1) est muni d'une macrostructuration, de préférence sous forme de cannelures (8) sur le pourtour périphérique.

19. Dispositif selon une des revendications 1 à 18, **caractérisé en ce que** le passage (2) de l'élément d'ostéosynthèse (1) présente, vers son côté inférieur (6) et de préférence également vers son côté supérieur (5), un rétrécissement de section transversale (7) pour empêcher que l'insert (10) ne tombe ou ne soit éjecté par pression.

20. Dispositif selon la revendication 19, **caractérisé en ce que** le rétrécissement de section transversale (7) du passage (2) et l'insert (10) réalisé en tant ressort à spirale sont adaptés l'un à l'autre de telle sorte que l'insert (10) peut être mis en place dans la section transversale (4) par un rétrécissement temporaire de son diamètre le plus grand.

21. Dispositif selon une des revendications 1 à 20, **caractérisé en ce que** la surface enveloppante extérieure (13) de l'insert (10) est réalisée de façon convexe.

22. Dispositif selon une des revendications 1 à 21, **caractérisé en ce que** le passage (2) de l'élément d'ostéosynthèse (1) est réalisé de façon concave.

23. Dispositif selon une des revendications 1 à 22, **caractérisé en ce que**
A) l'élément d'ostéosynthèse (1), au moins dans la zone de son passage (2), et
B) l'insert (10), au moins dans la zone de sa surface enveloppante extérieure (13),
C) sont composés de différents matériaux, de préférence de matériaux ayant des duretés différentes.

24. Dispositif selon une des revendications 1 à 23, **caractérisé en ce que** l'élément d'ostéosynthèse (1) est réalisé en matière plastique, de préférence une matière plastique renforcée, et **en ce que** l'insert (10) est métallique.

25. Dispositif selon une des revendications 1 à 24, **caractérisé en ce que** la hauteur de l'insert (10), mesurée dans la direction de son axe longitudinal (12), est plus petite que la hauteur du passage (2) dans la direction de son axe central (3).

26. Dispositif selon la revendication 25, **caractérisé en ce que** la hauteur de l'insert (10) se situe dans la plage de 40 % - 85 %, de préférence de 45 % à 65 %, de la hauteur du passage (2).

27. Dispositif selon une des revendications 1 à 26, **caractérisé en ce qu'**il comprend un élément de fixation osseuse (20) longitudinal, de préférence sous forme de vis à os avec une tige filetée (21) destinée à l'ancrage dans l'os, un axe de vis (23), et une tête de vis (22) qui est destinée à l'introduction dans le passage (11) central de l'insert (10) et qui correspond essentiellement à la forme du passage (11) de l'insert (10).

28. Dispositif selon la revendication 27, **caractérisé en ce que** la section transversale placée de façon orthogonale à l'axe de vis (23) rétrécit à travers la tête de vis (22) vers la tige filetée (21), de préférence sous forme de cône.

29. Dispositif selon la revendication 27 ou 28, **caractérisé en ce que** la tête de vis (22) est munie d'un filet extérieur (24).

30. Dispositif selon une des revendications 1 à 29, **caractérisé en ce que**
A) l'élément d'ostéosynthèse (1) est une plaque osseuse ;
B) le côté inférieur (6) convient pour s'appuyer contre l'os.

31. Dispositif selon une des revendications 1 à 29, **caractérisé en ce que** l'élément d'ostéosynthèse (1) est une vis pédiculaire ou un crochet pédiculaire.

32. Dispositif selon une des revendications 1 à 29, **caractérisé en ce que** l'élément d'ostéosynthèse (1) est un fixateur externe.

33. Dispositif selon une des revendications 1 à 29, **caractérisé en ce que** l'élément d'ostéosynthèse (1) est un implant intervertébral.

34. Dispositif selon une des revendications 29 à 33, **caractérisé en ce que** le diamètre du fil à ressort du ressort à spirale réalisé en tant qu'insert (10) est plus petit ou de même dimension que l'intervalle entre deux pointes filetées du filet extérieur (24) de la tête de vis (22).

35. Dispositif selon une des revendications 1 à 34, **caractérisé en ce que** la section transversale du fil à ressort du ressort à spirale réalisé en tant qu'insert (10) est non ronde, de préférence carrée ou rhombique.

36. Dispositif selon une des revendications 29 à 35, **caractérisé en ce que** la pente des spires du ressort à spirale réalisé en tant qu'insert (10) correspond essentiellement à la pente du filet extérieur (24) de la tête de vis (22).

37. Dispositif selon une des revendications 19 à 36, **caractérisé en ce que** le diamètre du fil à ressort du ressort à spirale est plus grand que le rétrécissement de section transversale (7) radial du passage (2).
